# EUROPÄISCHE PATENTANMELDUNG

(11) **EP 3 165 510 A1**
(43) Veröffentlichungstag der Anmeldung: **10.05.2017**
(21) Anmeldenummer: 15192735.7
(22) Anmeldetag: 03.11.2015
(51) Int. Cl.: C02F 1/32, G01N 15/02, G01N 15/06, G01N 15/10, G01N 33/18, F04D 15/02, F04D 1/00, F04D 13/06

(54) **KREISELPUMPENAGGREGAT**

(71) Anmelder: Grundfos Holding A/S, 8850 Bjerringbro (DK)
(72) Erfinder: Dahlqvist, Mathis, 7100 Vejle (DK); Guldbæk Smith, Christian, 8541 Skødstrup (DK); Iversen, Kåre, 8870 Langå (DK)
(74) Vertreter: Patentanwälte Vollmann & Hemmer

(57) **Zusammenfassung**

Das Kreiselpumpenaggregot weist einen Elektromotor (2) auf, der eine Kreiselpumpe (1) antreibt und ein Elektronikgehäuse (8) aufweist, in dem nicht nur die Steuerelektronik (10, 11) für Pumpe und Motor, sondern auch eine Vorrichtung zum Erfassen von Partikeln in Flüssigkeit integriert ist, die über einen Bypass (6, 7) der Förderleitung (3) gespeist wird.

## Beschreibung

Die Erfindung betrifft ein Kreiselpumpenaggregot mit den im Oberbegriff des Anspruchs 1 angegebenen Merkmalen. Die Erfindung betrifft darüber hinaus ein Verfahren zum Ansteuern eines solchen Kreiselpumpenaggregats.

Kreiselpumpenaggregate zählen in mannigfaltigen Bauarten zum Stand der Technik. Sie dienen zum Fördern von Flüssigkeiten, es gibt sie in einstufiger oder mehrstufiger Ausbildung, in Inline- oder auch anderer Ausführung. Gemeinsames Konstruktionsmerkmal ist mindestens ein Elektromotor, welcher mindestens eine Kreiselpumpe antreibt sowie eine Steuerelektronik. Dabei umfasst die Steuerelektronik bei modernen Kreiselpumpenaggregaten typischerweise einen elektronischen Drehzahlsteller, zum Beispiel einen Frequenzumrichter, mit dem die Motordrehzahl und damit die Drehzahl der Pumpe in weiten Grenzen variierbar ist. Kreiselpumpenaggregate im Sinne der vorliegenden Erfindung sind auch solche, bei denen zwei oder mehr Elektromotoren mit jeweils davon angetriebenen Kreiselpumpen vorgesehen sind, z.B. Doppelpumpen, die eine gemeinsame elektronische Steuerung und räumliche Anordnung aufweisen.

Pumpenaggregate dieser Art sind zur Förderung von Flüssigkeiten ausgelegt. Insbesondere bei der Trinkwasserförderung aber auch bei Förderung anderer Flüssigkeiten kann es erforderlich sein, die Qualität der Flüssigkeit zu erfassen und zu überwachen. So ist es für die Trinkwasserqualität wichtig zu wissen, welche Art und welche Menge von Bakterien oder anderen Partikeln vorhanden sind. Aber auch im Bereich der Verfahrenstechnik kann es erforderlich sein, die einem Prozess zugeführte Flüssigkeit hinsichtlich qualitativer/quantitativer Partikelbelastung zu überwachen. Solche Überwachungsverfahren, insbesondere auch Vorrichtungen, mit denen dies weitgehend automatisierbar möglich ist, zählen inzwischen zum Stand der Technik. Aus EP 2 469 264 A1 ist es bekannt, eine Flüssigkeitsprobe in einer optischen Analysevorrichtung zu durchstrahlen und Art und Anzahl der in der Probe befindlichen Partikel zu bestimmen.

Die Vorrichtung nach EP 2 469 264 A1 kann diese Bestimmung allerdings nur bei quasi ruhender Flüssigkeit vornehmen, weshalb die Einrichtung zur Eingliederung in ein Leitungssystem mit Durchfluss eher ungeeignet erscheint. Ein weiteres Problem ist die Anordnung einer solchen Vorrichtung in einer Anlage, da die Vorrichtung hoch empfindliche optische Messeinrichtungen enthält.

Gemäß der Erfindung ist vorgesehen, ein gattungsgemäßes Kreiselpumpenaggregat mit einer Vorrichtung zum Erfassen von Partikeln in Flüssigkeiten auszubilden. Dabei sind gemäß der Erfindung in dem Kreiselpumpenaggregat mindestens Mittel zum Zuführen von Förderflüssigkeit zur Vorrichtung vorgesehen, um zumindest einen Teilstrom der Förderflüssigkeit zur Vorrichtung hin abzweigen zu können und quasi kontinuierlich oder bei Bedarf eine Probenentnahme von der Förderflüssigkeit zur Vorrichtung zu gewährleisten.

Grundgedanke der vorliegenden Erfindung ist es, eine Vorrichtung zum Erfassen von Partikeln in Flüssigkeit in ein Kreiselpumpenaggregat zu integrieren und dabei, auch wenn die Vorrichtung nicht kontinuierlich arbeitet, gewisse Synergien zu schaffen, insbesondere in Weiterbildung das Kreiselpumpenaggregot zur Ausführung eines weiter unten noch erläuterten erfindungsgemäßen Verfahrens auszugestalten.

Das erfindungsgemäße Kreiselpumpenaggregat, welches insbesondere ein Trinkwasserförderpumpenaggregat ist, weist mindestens einen Elektromotor und mindestens eine elektromotorisch angetriebene Kreiselpumpe auf sowie eine Steuerelektronik. Gemäß der Erfindung ist das Aggregat mit einer Vorrichtung zum Erfassen von Partikeln in Flüssigkeit ausgestattet und weist darüber hinaus zumindest Mittel zum Zuführen von Förderflüssigkeit zu dieser Vorrichtung auf. Das erfindungsgemäße Kreiselpumpenaggregot ist somit dazu ausgebildet, nicht nur Flüssigkeit zu fördern, sondern darüber hinaus auch Teile dieser Flüssigkeit quantitativ und/oder qualitativ hinsichtlich von in der Flüssigkeit befindlichen Partikeln zu untersuchen. Dabei sind Partikel im Sinne der vorliegenden Erfindung typischerweise Bakterien aber auch Schwebstoffe oder Sinkstoffe ähnlicher Größe.

Das Kreiselpumpenaggregot weist typischerweise einen Elektromotor und eine davon angetriebenen ein- oder mehrstufige Kreiselpumpe auf. Es kann jedoch auch als Doppelpumpe ausgebildet sein. Dabei kann ein Aggregat im Sinne der vorliegenden Erfindung auch die Anordnung und Verschaltung mehrerer jeweils von einem Elektromotor angetriebenen Kreiselpumpen sein, die eine gemeinsame Steuerelektronik aufweisen oder zumindest einen gemeinsamen Steuerelektronikteil, der einer solchen Vorrichtung zugeordnet ist, beispielsweise eine Anordnung wie sie von der Anmelderin unter Grundfos Multi Hydro angeboten wird.

Die erfindungsgemäße Ausgestaltung des Kreiselpumpenaggregats mit einer solchen Vorrichtung hat den Vorteil, dass gewährleistet ist, dass die Vorrichtung an zentraler Stelle, nämlich dort wo die Flüssigkeit gefördert wird, angeordnet ist und dass im weiteren die Steuerelektronik für die Vorrichtung einerseits und für die Steuerung des Elektromotors bzw. der damit verbundenen Kreiselpumpen andererseits zumindest räumlich weitgehend zusammengeführt ist. Das Kreiselpumpenaggregat ist eine bauliche Einheit, im Wesentlichen bestehend aus Kreiselpumpe, Steuerung, Elektromotor und Vorrichtung.

Hierdurch ergeben sich Synergieeffekte wie beispielsweise die Nutzung einer gemeinsamen Versorgungsnetzanbindung, die gemeinsame Nutzung von Netzteilen, die gemeinsame Nutzung von Mikroprozessoren, Speichern und dergleichen digitalen Bausteinen sowie von Kommunikationsbausteinen mit LAN-Anbindung, WLAN-Anbindung, Bluetooth-Anbindung, Infrarotanbindung oder dergleichen. Schließlich bildet in einer Anlage das Kreiselpumpenaggregot stets eine Art Fundament, sei es, dass durch die Massekonzentration im Rohrsystem eine Art ruhender Pol gebildet ist oder sei es, dass das Kreiselpumpenaggregat selbst durch ein Fundament abgestützt ist. Dies ist insbesondere für die Anordnung der Vorrichtung von besonderem Vorteil, da es sich hierbei um ein empfindliches optisches Analysesystem handelt. Schließlich kann die Zuführung von Förderflüssigkeit zur Vorrichtung am Pumpenaggregat besonders einfach und kostengünstig realisiert werden, indem entweder entsprechende Kanäle in Pumpenbauteilen vorgesehen werden oder eine Anbindung über Leitungen erfolgt. In beiden Fällen ist der Bauaufwand gering und kann fabrikmäßig erfolgen, ist also nicht vor Ort zu verrohren.

Zum Aufbau der Vorrichtung wird ergänzend auf die unter Anmeldenummer 14186884.4 beim Europäischen Patentamt geführte Patentanmeldung der Anmelderin verwiesen, die eine solche Vorrichtung mit auswechselbarem Probenträger detailliert beschreibt.

Vorteilhafte Ausgestaltungen der Erfindung sind in den Unteransprüchen, der nachfolgenden Beschreibung und den Zeichnungen angegeben.

Besonders vorteilhaft ist es, wenn die Vorrichtung hydraulisch in einen Bypass des Förderwegs der Pumpe bzw. der Pumpen eingegliedert ist, da dann gegebenenfalls auf eine gesonderte Pumpe zur Förderung der Flüssigkeit von und zu der Vorrichtung verzichtet werden kann, zumindest wenn die Vorrichtung mit ihrer Zuführleitung druckseitig und mit einer gegebenenfalls vorhandenen Rückführleitung saugseitig am Pumpenaggregat, also gegenläufig zum Förderweg der Pumpe, angeschlossen ist.

Alternativ oder zusätzlich kann die Vorrichtung vorteilhaft ausgangsseitig eine Leitung zum Anschluss an einen Abfluss, insbesondere an eine Abwasserleitung oder an einen Auffangbehälter ausweisen, wenn verhindert werden soll, dass die in der Vorrichtung untersuchte Flüssigkeit wieder in den Förderweg gelangen soll. Es ist auch denkbar, die untersuchte Flüssigkeit entsprechenden Probenbehältern zuzuführen und zu archivieren.

Um das Zuführen und Abführen von Flüssigkeit zur Vorrichtung hin und von der Vorrichtung weg steuern zu können, ist gemäß einer Weiterbildung vorgesehen, ein elektrisch steuerbares Absperrventil vorteilhaft eingangsseitig und ausgangsseitig der Vorrichtung vorzusehen. Damit kann die Vorrichtung automatisiert beschickt werden oder bei Empfang eines entsprechenden Steuerbefehls in der Steuerelektronik. Wenn das Kreiselpumpenaggregat, wie dies häufig der Fall ist, als Baueinheit, im Wesentlichen bestehend aus einer Kreiselpumpe, einem diese antreibenden Elektromotor und einem Elektronikgehäuse, welches in der Regel am Motorgehäuse befestigt und angeordnet ist, ausgebildet ist, dann ist es besonders vorteilhaft, wenn dieses Elektronikgehäuse zumindest Teile der Vorrichtung oder die Vorrichtung selbst aufnimmt. Dies ist auch deshalb vorteilhaft, weil dann die mechanischen und hydraulischen Baugruppen, nämlich Elektromotor und Pumpe, weitgehend unverändert belassen werden können, hier also Baugruppen Verwendung finden können, die auch bei anderen Kreiselpumpenaggregaten eingesetzt werden und lediglich das Elektronikgehäuse entsprechend anzupassen ist. Letzteres ist sowohl hinsichtlich des inneren Aufbaus als auch hinsichtlich der äußeren räumlichen Erstreckung weitgehend frei gestaltbar.

Vorteilhaft wird das erfindungsgemäße Kreiselpumpenaggregot nicht nur zur Untersuchung eines Teils der Förderflüssigkeit herangezogen, sondern in Weiterbildung der Erfindung auch zu der Behandlung der Förderflüssigkeit, insbesondere wenn sich bei der Untersuchung in der Vorrichtung herausstellt, dass ein solcher Behandlungsbedarf besteht, beispielsweise wenn die Bakterienbelastung der Förderflüssigkeit, insbesondere des Trinkwassers, zu hoch ist oder eine bestimmte Bakterienart in zu hoher Konzentration festgestellt wird. Dann ist gemäß einer vorteilhaften Ausgestaltung der Erfindung vorgesehen, ein drehzahlsteuerbares Kreiselpumpenaggregot drehzahlmäßig in Abhängigkeit der Art und/oder Anzahl der erfassten Partikel anzusteuern. Es können also in der Steuerelektronik des Kreiselpumpenaggregats Drehzahleingriffe vorgesehen sein, die beispielsweise die Pumpe mit erhöhter Drehzahl ansteuern, wenn in der Vorrichtung das Überschreiten einer vorbestimmten Anzahl von Partikeln pro Volumeneinheit registriert wird oder wenn bestimmte Bakterien erfasst oder in einer unzulässig hohen Menge erfasst werden. Dabei kann gemäß der Erfindung vorgesehen sein, dass die Pumpe mit einer Drehzahl oder einer Drehzahlfolge angesteuert wird, welche insbesondere im Saugbereich der Pumpe Kavitation hervorruft, um zumindest einen Teil dieser Bakterien abzutöten oder beispielsweise große Partikel in kleinere zu zerschlagen. Alternativ oder zusätzlich kann im Pumpenaggregat eine Förderflüssigkeitsbehandlungseinrichtung, beispielsweise durch UV-, Ultraschall-, Röntgenstrahlung oder dergleichen vorgesehen sein, die bei Überschreiten eines vorbestimmten Grenzwertes innerhalb der Vorrichtung aktiviert bzw. bei Unterschreiten wieder deaktiviert wird.

Gemäß einer Weiterbildung der Erfindung kann die Steuerelektronik des erfindungsgemäßen Kreiselpumpenaggregats so ausgebildet sein, dass sie den Antrieb der Pumpe umsteuert, insbesondere kavitierend ansteuert, sobald mittels der Vorrichtung eine Anzahl von Partikeln pro Volumeneinheit der Förderflüssigkeit und/oder eine Art von Partikeln erfasst wird, die einen zuvor festgelegten Wert quantitativen und/oder qualitativen Grenzwert erreicht. Anstelle oder zusätzlich zur Umsteuerung des Pumpenantriebs kann eine im Pumpenaggregat integrierte oder im Förderweg unmittelbar vor oder nach der Pumpe angeordnete Flüssigkeitsbehandlungseinrichtung aktiviert werden, insbesondere bei der Trinkwasserförderung aber auch bei der Förderung von Flüssigkeiten in industriellen Verfahren.

Es ist besonders vorteilhaft, wenn die Steuerelektronik so ausgebildet ist, dass sie in vorbestimmbaren zeitlichen Abständen durch öffnende Ventilansteuerung der Vorrichtung eine Flüssigkeitsmenge vom Förderstrom zuführt und die Vorrichtung zum Erfassen von Partikeln ansteuert. Mit einer solchen Ausbildung ist eine quasi kontinuierliche Überwachung der Förderflüssigkeit möglich. Die Erfassung von Partikeln durch die Vorrichtung kann zeitmäßig gleichzeitig mit einer Pumpaktivität der Kreiselpumpe geschehen. In einer Variante der Erfindung wird die Pumpe automatisch gestoppt während die Vorrichtung Partikel erfasst oder die Drehzahl der Pumpe wird automatisch auf eine Drehzahl geregelt, die keine oder nur niedrige Vibration erzeugt. Die Drehzahl kann auch während des Erfassens konstant gehalten werden. Hierdurch wird die Flüssigkeit während des Erfassens beruhigt gehalten.

Insbesondere bei der Untersuchung von Trinkwasser ist es praktisch unvermeidbar, dass sich im Laufe der Zeit Ablagerungen ansammeln, beispielsweise Biofilm, Kalk oder dergleichen. Um hier sicherzustellen, dass die qualitativen/quantitativen Untersuchungen der Vorrichtung zuverlässig und mit der erforderlichen Genauigkeit stattfinden können, ist gemäß der Erfindung vorgesehen, die Vorrichtung mit einem Probenträger auszustatten, der zwar durchströmbar ist, in dem jedoch die eigentliche Untersuchung bei stehender/ruhender Flüssigkeit, also quasi stationär erfolgt. Dieser Probenträger ist gemäß der Erfindung vorteilhaft auswechselbar an der Vorrichtung angeordnet und zwar so, dass er von einer Gehäuseseite des Kreiselpumpenaggregats, insbesondere des Elektronikgehäuses - wenn die Vorrichtung dort integriert ist - zugänglich ist. Der Probenträger wird zweckmäßigerweise so angeordnet, dass er an einer gut zugänglichen Stelle des Kreiselpumpenaggregats angeordnet und schnell und einfach austauschbar ist.

Auch wenn das Kreiselpumpenaggregat, insbesondere die Pumpe mit dem Elektromotor, für die Vorrichtung ein gewisses ruhendes Fundament bildet, ist gemäß einer Weiterbildung der Erfindung vorgesehen, dass zumindest der mechanische/optische Teil der Vorrichtung schwingungsgedämpft innerhalb des Gehäuses, insbesondere des Elektronikgehäuses, angeordnet ist, um zu verhindern, dass die durch den Elektromotor erzeugten aber auch möglicherweise über das Rohrleitungssystem in das Aggregat eingebrachten Schwingungen auf die empfindlichen optischen/mechanischen Bauteile einen die Messung verschlechternden Einfluss ausüben.

Insbesondere wenn das Kreiselpumpenaggregot in eine Hochdruckleitung eingegliedert ist oder, wie dies insbesondere bei mehrstufigen Pumpen der Fall sein kann, die Pumpe mit hohem Druck fördert, ist es vorteilhaft, eingangsseitig der Vorrichtung Mittel zur Druckreduzierung vorzusehen, damit die Vorrichtung, insbesondere der Probenträger, nicht dem hohen Systemdruck ausgesetzt ist.

Gemäß einer vorteilhaften Weiterbildung der Erfindung sind jedoch diese Mittel zur Druckreduzierung in der Eingangsleitung der Vorrichtung entweder durch entsprechende Ansteuerung oder durch Vorsehen einer Bypassleitung deaktivierbar. Diese Deaktivierung wird vorteilhaft dazu genutzt, die Vorrichtung mit erhöhtem Druck durchzuspülen, um die bereits erwähnten Ablagerungen zu entfernen.

Zwar kann, wie eingangs erläutert, die Vorrichtung durch den Druck der Förderflüssigkeit beschickt werden, insbesondere wenn sie druckseitig der Pumpe angeschlossen ist, doch setzt dies stets voraus, dass die Pumpe fördert, und zwar mit dem erforderlichen Druck. Insoweit unabhängiger ist eine Ausgestaltung, bei welcher eine gesonderte Pumpe zum Zuführen und/oder Abführen der Förderflüssigkeit vorgesehen ist, welche vorzugsweise im Elektronikgehäuse angeordnet ist. Es kann sich hierbei ebenfalls um eine Kreiselpumpe, vorteilhaft jedoch um eine Verdrängerpumpe, beispielsweise eine Membranpumpe oder eine Impellerpumpe handeln. Mit einer solchen Pumpe ist unabhängig vom Systemzustand der Förderpumpe stets sichergestellt, dass eine entsprechende Menge der Förderflüssigkeit der Vorrichtung zu- bzw. aus dieser abgeführt wird.

Weiterhin ist es vorteilhaft, wenn die Vorrichtung eine Bestrahlungseinrichtung für die in der Vorrichtung befindliche oder aus der Vorrichtung herausgeführte Flüssigkeit aufweist. Eine solche Bestrahlungseinrichtung kann beispielsweise eine UV-Bestrahlungseinrichtung sein, welche sicherstellt, dass die aus der Vorrichtung herausgeführte Flüssigkeit nicht verkeimt ist. Auch eine solche Bestrahlungseinrichtung wird vorteilhaft innerhalb des Elektronikgehäuses angeordnet. Das Elektronikgehäuse bildet dabei zugleich ein Schutzgehäuse, welches verhindert, dass Strahlung der Bestrahlungseinrichtung nach außen gelangt.

Vorteilhaft weist die Steuerelektronik, die zumindest teilweise innerhalb des Elektronikgehäuses angeordnet ist, sowohl die Steuer- und Auswertelektronik der Vorrichtung als auch die Steuerelektronik der Pumpe auf, insbesondere die Drehzahlstellerelektronik, welche den Elektromotor ansteuert, der die Kreiselpumpe antreibt.

Dabei weist die Steuerelektronik vorteilhaft Mittel zum Speichern von Daten der Auswertelektronik sowie Mittel zum drahtgebundenen und/oder drahtlosen Übertragen von Daten der Auswertelektronik und/oder von gespeicherten Daten auf. Dabei können die Kommunikationsmittel zum Übertragen von Daten vorteilhaft sowohl zum Übertragen von Daten der Vorrichtung als auch zum Übertragen von Daten der Pumpensteuerung dienen. Insbesondere die digitalen Baugruppen der Steuerelektronik wie Prozessor, Arbeitsspeicher und Festspeicher können vorteilhaft sowohl für die Daten der Pumpensteuerung als auch für Daten der Vorrichtung genutzt werden. Hierdurch können zum Beispiel beim Einsatz heute üblicher Frequenzumrichterelektronik Teile der vorhandenen Elektronik für Steuerung und Auswertung der Vorrichtung mit benutzt werden. Insbesondere kann die komplette Ablaufsteuerung für die Vorrichtung davon übernommen werden.

Die Erfindung ist nachfolgend anhand von Ausführungsbeispielen näher erläutert. Es zeigen:
- Fig. 1: ein Blockdiagramm betreffend ein erfindungsgemäßes Kreiselpumpenaggregat mit einer Vorrichtung zum Erfassen von Partikeln in Flüssigkeiten,
- Fig. 2: eine mehrstufige Inline-Kreiselpumpe mit Elektromotor, Elektronikgehäuse und darin integrierter Vorrichtung in stark vereinfachter und perspektivischer Seitenansicht
- Fig. 3: das Pumpenaggregat gemäß Fig. 2 in perspektivischer Ansicht von einer anderen Seite,
- Fig. 4: das Pumpenaggregat gemäß Fig. 2 in Seitenansicht,
- Fig. 5: das Pumpenaggregat gemäß Fig. 2 in gegenüber Fig. 4 um 90° versetzter Seitenansicht,
- Fig. 6: in stark vereinfachter perspektivischer Ansicht ein Umwälzpumpenaggregat mit Vorrichtung zum Erfassen von Partikeln in Flüssigkeit,
- Fig. 7: das Umwälzpumpenaggregat gemäß Fig. 6 in anderer perspektivischer Darstellung,
- Fig. 8: eine Ansicht auf das Umwälzpumpenaggregat gemäß Fig. 6 von vorne,
- Fig. 9: eine Ansicht auf das Umwälzpumpenaggregat gemäß Fig. 6 von der Seite und
- Fig. 10: eine Ansicht auf das Umwälzpumpenaggregat gemäß Fig. 6 von der anderen Seite.

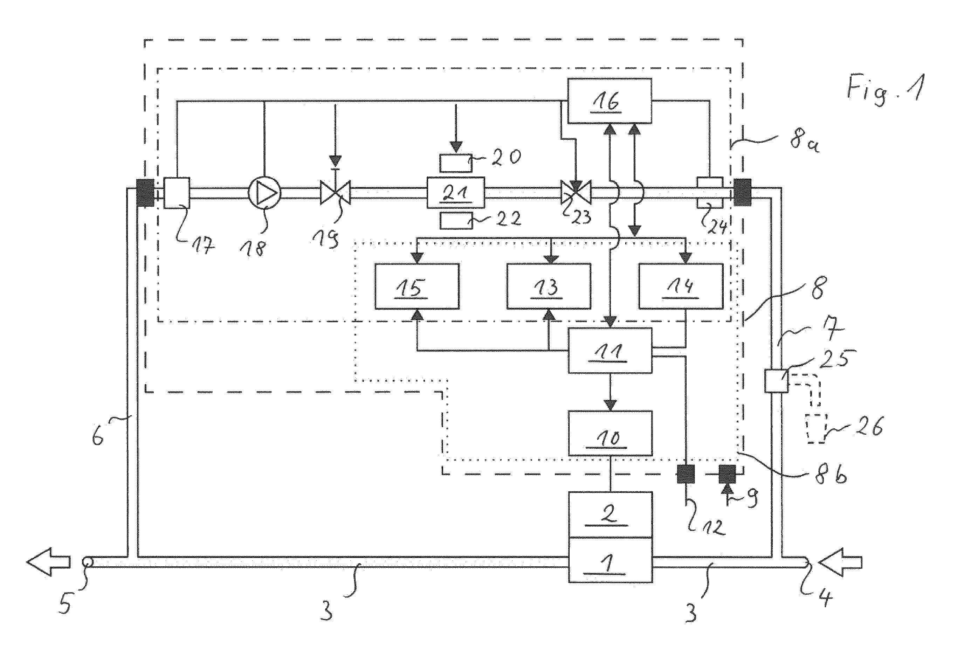

Der grundsätzliche Aufbau eines Kreiselpumpenaggregats gemäß der Erfindung ist anhand der Fig. 1 dargestellt. Das Kreiselpumpenaggregat weist eine Kreiselpumpe 1 auf, die von einem Elektromotor 2 angetrieben ist. Die Kreiselpumpe 1 fördert durch eine Hauptförderleitung 3, deren Eingang in Fig. 1 mit 4 und deren Ausgang mit 5 gekennzeichnet ist. Die Hauptförderleitung 3 führt eingangsseitig zur Saugseite der Pumpe 1, deren Druckseite über die Leitung mit dem Ausgang 5 verbunden ist.

Parallel zur Kreiselpumpe 1 ist eine Bypassleitung 6, 7 vorgesehen, die sich in einen druckseitigen Teil 6 und einen saugseitigen Teil des Bypasses 7 aufteilt. Diese Bypassleitung 6, 7 führt in ein Elektronikgehäuse 8, welches am Elektromotor 2 angeordnet und befestigt ist und welches einerseits eine Art Klemmenkasten bildet, in das die Wicklungen des Motors herausgeführt und kontaktiert sind und in das andererseits eine elektrische Netzversorgung 9 geführt ist. Innerhalb dieses Elektronikgehäuses 8 ist eine Vorrichtung zum Erfassen von Partikeln in Flüssigkeit integriert, deren Aufbau im Wesentlichen in der europäischen Patentanmeldung 14186884.4 der Anmelderin beschrieben ist, auf die hiermit explizit verwiesen wird. In dem Elektronikgehäuse 8 sind also sowohl der mechanische als auch der elektronische Teil der vorgenannten Vorrichtung sowie die Steuer- und Regelelektronik des Motors 2 bzw. der daran angeschlossenen Pumpe 1 angeordnet.

In Fig. 1 ist die Motorsteuerung mit 10 gekennzeichnet, sie weist einen Frequenzumrichter auf, dessen Ausgang die Wicklungen des Elektromotors 2 direkt beaufschlagt. Der Motorsteuerung 10 vorgeschaltet ist eine Pumpensteuerung 11, welche über eine Input/Output-Schnittstelle 12 mit Sensoren innerhalb der Pumpe 1 kommuniziert.

Weiterhin sind innerhalb des Elektronikgehäuses 8 ein Display 13, eine Bedienoberfläche 14 sowie eine Kommunikationsschnittstelle 15 vorgesehen, die sowohl mit der Pumpensteuerung 11 als auch mit einer Steuerelektronik 16 in Verbindung stehen, welche Teil der Vorrichtung zum Erfassen von Partikeln ist. Bei dem Display 13 handelt es sich um ein LCD Display, es kann jedoch hier auch grundsätzlich jede andere geeignete Signal- oder Anzeigevorrichtung Verwendung finden. Die Bedienoberfläche 14 ist als Folientastatur ausgebildet, auch hier kann jede andere geeignete Signaleingabequelle verwendet werden. Als Kommunikationsschnittstelle 15 ist ein LAN-Anschluss sowie ein WLAN-Senderempfänger vorgesehen. Auch hier kann grundsätzlich jede andere geeignete Kommunikationsschnittstelle Verwendung finden.

Die Vorrichtung zum Erfassen von Partikeln in Flüssigkeit wird über den druckseitig von der Hauptförderleitung 3 abgezweigten Teil der Bypassleitung 6 gespeist. Innerhalb der Vorrichtung ist zunächst ein steuerbarer Druckminderer 17 vorgesehen, dem eine Förderpumpe 18 nachgeschaltet ist, welche über ein steuerbares Absperrventil 19 mit der eigentlichen Analyseeinrichtung in Verbindung steht. Die Analyseeinrichtung besteht im Wesentlichen aus einer Leuchtquelle 20, einer Aufnahmeeinrichtung 21 für einen Probenträger und einem optischen Sensor 22 in Form einer Sensormatrix, dessen Daten in der Steuerelektronik 16 ausgewertet, gespeichert und weitergeleitet werden. Ausgangsseitig ist die Aufnahmeeinrichtung 21 für den Probenträger über ein elektrisch steuerbares Absperrventil 23 mit einer UV-Behandlungseinrichtung 24 verbunden, deren Ausgang mit dem saugseitigen Teil 7 der Bypassleitung verbunden ist, welche saugseitig der Pumpe, am Eingang 4 in die Hauptförderleitung 3 mündet. Die Förderpumpe 18 kann in Systemen mit relativ hohem Druck weggelassen werden.

Innerhalb dieser Leitung 7 ist eine Abzweigung 25 vorgesehen, über welche die aus der Analyseeinrichtung 20, 21 und 22 kommende Flüssigkeit, wenn diese nicht der Hauptförderleitung 3 zugeführt werden soll, einem Sammelbehälter 26 zuführbar ist, der hier stellvertretend für beispielsweise eine Abflussleitung, einen Auffangbehälter oder ein Archiviersystem steht, in welchem die aus der Analyseeinrichtung 20, 21 und 22 kommende Flüssigkeit aufgefangen und/oder archiviert werden kann. In einer Variante der Erfindung wird nicht oder nicht nur Flüssigkeit in der Bypassleitung abgezweigt, sondern auch die eigentlich zu fördernde Flüssigkeit in der Haupttförderleitung 3. Im Falle eines Bakterienalarms kann die Steuerelektronik ein Umschaltventil in der Hauptförderleitung (Umschaltventil in der Zeichnung nicht gezeigt) so umschalten, dass die bakterieninfizierte Flüssigkeit in eine andere Leitung umgeleitet wird. Dieses Umschaltventil kann selbstständig in der Hauptleitung eingebaut sein, oder ein Teil des Kreiselpumpenaggregats sein, z. B. im Pumpengehäuse eingebaut.

Die Vorrichtung zum Erfassen von Partikeln in Flüssigkeit, wie sie anhand von Fig. 1 dargestellt ist, weist einen steuerbaren Druckminderer 17 auf, eine Förderpumpe 18, eine Analyseeinrichtung 20 - 22, die über zwei Absperrventile 19 und 23 in die Leitung 6, 7 eingegliedert ist, und eine nachgeschaltete UV-Behandlungseinrichtung 24 auf. Die gesamte Ablaufsteuerung, Auswertung, Speicherung und Weiterleitung von Daten erfolgt in der Steuerelektronik 16. Die Bedienung der Vorrichtung erfolgt mit Ausnahme des Auswechselns des in der Aufnahmeeinrichtung 21 befindlichen Probenträgers über die Bedienoberfläche 14, über welche auch die Bedienung der Pumpensteuerung erfolgt. Die Anzeige der Vorrichtung erfolgt über das Display 13, über welches auch die Anzeige der Pumpensteuerung erfolgt. Weiterhin nutzen sowohl die Steuerelektronik 16 als auch die Pumpensteuerung 11 dieselben Kommunikationsschnittstellen 15. In Fig. 1 nicht im Einzelnen dargestellt ist die gemeinsame Nutzung der digitalen Komponenten der Steuerelektronik 16 und der Pumpensteuerung 11, nämlich Prozessor, Arbeitsspeicher und Festspeicher. Die zum Erfassen von Partikeln in Flüssigkeit innerhalb des Elektronikgehäuses 8 vorgesehenen Komponenten sind mit 8a gekennzeichnet, die für die Pumpen-/Motorsteuerung vorgesehenen mit 8b. Deutlich sichtbar ist hierbei der sich überschneidende Bereich betreffend Display 13, Bedienoberfläche 14 und Kommunikationsschnittstellen 15, die sowohl der Vorrichtung als auch der Motor-/Pumpensteuerung 10, 11 zugeordnet sind.

In die Vorrichtung zum Erfassen von Partikeln in Flüssigkeit gelangt die Flüssigkeit über die Bypassleitung 6 zunächst in den Druckminderer 17, der durch die Steuerelektronik 16 steuerbar ist und sicherstellt, dass die Flüssigkeit einen vorbestimmten Druck nicht überschreitet. Über die Förderpumpe 18 gelangt die zu analysierende Flüssigkeit bei geöffneten Ventilen 19 und 23 in den in der Aufnahmeeinrichtung 21 befindlichen Probenträger. Nachfolgend werden die Ventile 19 und 23 geschlossen, wonach die Analyse der in der Analyseeinrichtung 20 - 22 befindlichen Flüssigkeitsprobe stattfindet, deren Ablaufsteuerung und Auswertung in der Steuerelektronik 16 erfolgt. Nach erfolgter Analyse kann durch Öffnen der Ventile 19 und 23 die in der Analyseeinrichtung befindliche Flüssigkeit durch die UV-Behandlungseinrichtung 24 geleitet werden, die in der die in der Analyseeinrichtung 20 - 22 untersuchte Flüssigkeitsprobe gegebenenfalls einer UV-Behandlung unterzogen wird, um darin befindliche Bakterien abzutöten. Die UV-Behandlung erfolgt zweckmäßigerweise nur bei erhöhter oder spezieller Bakterienbelastung der Flüssigkeitsprobe. Die insoweit bereinigte Flüssigkeit kann dann über den Teil 7 der Bypassleitung wieder eingangsseitig dem Förderstrom der Hauptförderleitung 3 zugeführt werden, wenn eine Flüssigkeitsführung im geschlossenen Kreislauf gewünscht wird. Es ist auch möglich, die UV-Bestrahlungseinrichtung 24 in der Analyseeinrichtung 20 - 22 anzubringen und direkt die Flüssigkeit zu bestrahlen.

Zum Zwecke der Reinigung kann ein in der Aufnahmeeinrichtung 21 befindlicher Probenträger auch dadurch gereinigt werden, dass die Ventile 19 und 23 geöffnet und der Druckminderer 17 funktionslos gesteuert wird. Dann strömt die Flüssigkeit über die Bypassleitung 6, 7 mit dem Förderdruck der Pumpe 1 im Rücklauf durch die Vorrichtung, wobei die Pumpe 18 stromlos geschaltet ist und durchströmt wird. Wenn als Pumpe 18 keine Kreiselpumpe sondern eine Verdrängerpumpe eingesetzt wird, ist an dieser Stelle für eine Bypassleitung zu sorgen, die zum Zwecke des Spülvorgangs freigeschaltet werden kann.

Weiterhin ist vorgesehen, dass mittels der Steuerelektronik 16, wenn beispielsweise eine erhöhte oder bestimmte Bakterienbelastung in der Flüssigkeit festgestellt wird, die Pumpensteuerung 11 ein Signal erhält, welches diese veranlasst, die Drehzahl des Elektromotors 2 so anzusteuern, dass die Pumpe 1 in ihrem Saugbereich kavitiert, das heißt gezielt Kavitation erzeugt und hierdurch einen Bakterien abtötenden Effekt auf die Förderflüssigkeit auszuüben. Dabei wird durch diesen kavitierenden Effekt der Pumpe 1 nicht nur die in die Hauptförderleitung 3 rückgeführte Probenflüssigkeit, sondern der gesamte Hauptförderstrom behandelt.

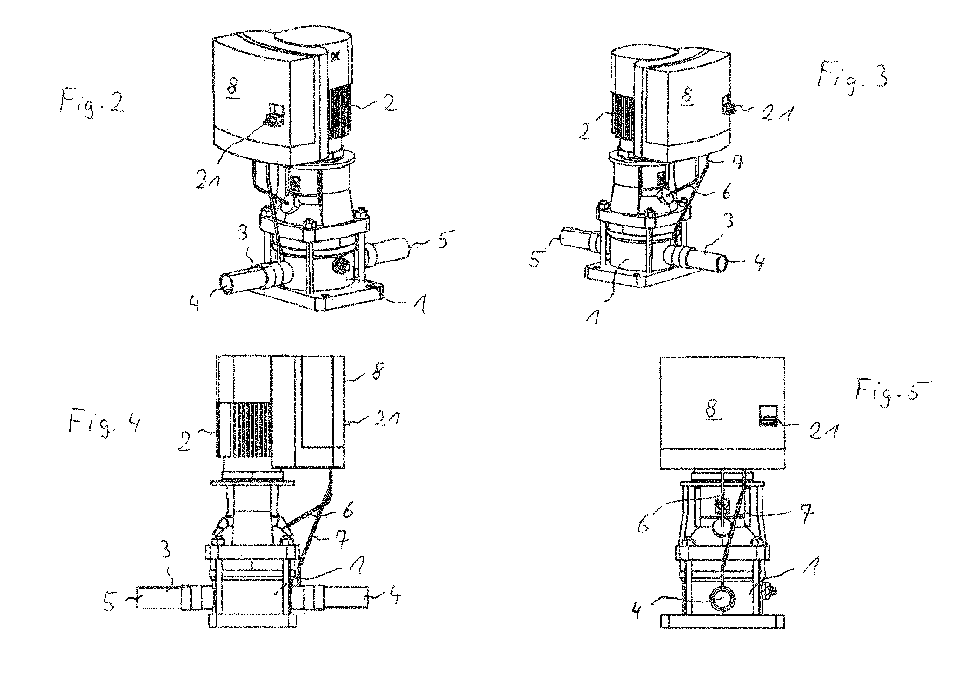

Anhand der Fig. 2 - 5 ist beispielhaft dargestellt, wie die anhand der Fig. 1 schematisch dargestellte Verknüpfung von Baugruppen in einem Pumpenaggregat integriert werden kann, wie dies beispielsweise durch die Baureihe CRE der Anmelderin (geregelte vertikale mehrstufige Kreiselpumpen) bekannt ist. Die baulichen Änderungen gegenüber einem Standardpumpenaggregat sind vergleichsweise gering. Es sind hier pumpenseitig lediglich die Leitungen 6 und 7 für die Bypassleitung herauszuführen, was keiner großen baulichen Veränderung bedarf, da, wie die Fig. 2 - 5 verdeutlichen, der saugseitige Teil 7 nahe dem Anschlussstutzen und der druckseitige Teil 6 aus dem Pumpenkopf herausgeführt sind. Das Elektronikgehäuse 8 ist von der Baugröße her deutlich vergrößert, da es die Vorrichtung zum Erfassen von Partikeln aufnehmen muss, das heißt die Baugruppen 17 - 24. In den Darstellungen gemäß den Fig. 2 - 5 deutlich erkennbar ist die am Elektronikgehäuse von vorne zugängliche Aufnahmeeinrichtung 21 für den Probenträger, der von vorne auswechselbar ist.

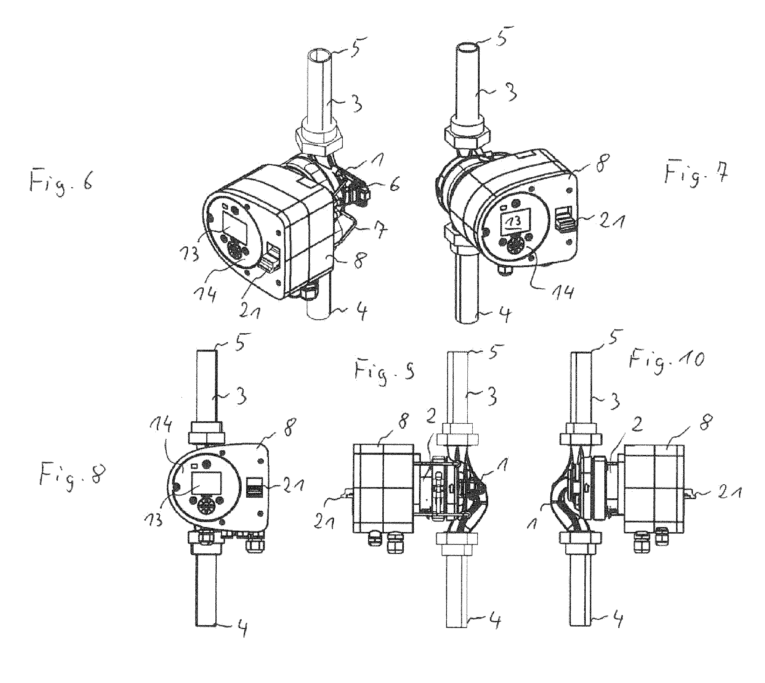

Anhand der Fig. 6 - 10 ist beispielhaft dargestellt, wie ein Elektronikgehäuse 8 einer üblichen Umwälzpumpe ausgestaltet werden kann, um die Baugruppen 17 - 24 mit aufnehmen zu können. Die Leitungen 6 und 7 sind bei dieser einstufigen Pumpe gemäß den Fig. 6 - 10 außerhalb des Pumpengehäuses geführt. Die Leitungen 6 und 7 können aber auch innerhalb des Pumpengehäuses durch Kanäle gebildet sein, welche über den Boden des Elektronikgehäuses in dieses hineingeführt sind.

Es sei darauf hingewiesen, dass die für die Fig. 4 - 10 verwendeten Bezugszeichen Bauteile gleicher Funktion betreffen, auch wenn deren konstruktive Ausgestaltung einander nicht entspricht.

### Bezugszeichenliste

- 1: - Kreiselpumpe
- 2: - Elektromotor
- 3: - Hauptförderleitung
- 4: - Eingang
- 5: - Ausgang
- 6: - druckseitiger Teil des Bypasses
- 7: - saugseitiger Teil des Bypasses
- 8: - Elektronikgehäuse
- 9: - Netzversorgung
- 10: - Motorsteuerung
- 11: - Pumpensteuerung
- 12: - I/O- Schnittstelle
- 13: - Display
- 14: - Bedienoberfläche
- 15: - Kommunikationsschnittstellen
- 16: - Steuerelektronik der Vorrichtung
- 17: - steuerbarer Druckminderer
- 18: - Förderpumpe
- 19: - elektrisch steuerbares Absperrventil
- 20: - Leuchtquelle
- 21: - Aufnahmeeinrichtung für Probenträger
- 22: - optischer Sensor
- 23: - elektrisch steuerbares Absperrventil
- 24: - UV-Behandlungseinrichtung
- 25: - Abzweigung
- 26: - Sammelbehälter
- 8a: - Teil der Vorrichtung
- 8b: - Teil der Motor-/Pumpensteuerung

## Patentansprüche

1. Kreiselpumpenaggregat, insbesondere zum Ausführen eines Verfahrens nach einem der Ansprüche 16 bis 18, insbesondere Trinkwasserförderpumpenaggregat, mit mindestens einem Elektromotor (2), mit mindestens einer elektromotorisch angetriebenen Kreiselpumpe (1) und mit einer Steuerelektronik (10, 11), **dadurch gekennzeichnet, dass** das Aggregat eine Vorrichtung zum Erfassen von Partikeln in Flüssigkeit (20 - 22) und Mittel (6, 7) zum Zuführen von Förderflüssigkeit (20 - 22) zur Vorrichtung aufweist.

2. Kreiselpumpenaggregot nach Anspruch 1, **dadurch gekennzeichnet, dass** die Vorrichtung (20 - 22) in einen Bypass (6, 7) des Förderwegs (3) der Pumpe/n (1) eingegliedert ist.

3. Kreiselpumpenaggregot nach Anspruch 2, **dadurch gekennzeichnet, dass** die Vorrichtung (20 - 22) ausgangsseitig eine Leitung zum Anschluss an einen Abfluss, insbesondere an eine Abwasserleitung, oder an einen Auffangbehälter (26) aufweist.

4. Kreiselpumpenaggregot nach einem der vorhergehenden Ansprüche, **dadurch gekennzeichnet, dass** ein elektrisch steuerbares Absperrventil (19, 23) eingangsseitig und/oder ausgangsseitig der Vorrichtung (20 - 22) vorgesehen ist.

5. Kreiselpumpenaggregot nach einem der vorhergehenden Ansprüche, **dadurch gekennzeichnet, dass** die Steuerelektronik (10, 11, 16) in einem Elektronikgehäuse (8) angeordnet ist, welches zumindest Teile der Vorrichtung (20 - 22) aufnimmt und welches am Motorgehäuse und/oder Pumpengehäuse befestigt ist.

6. Kreiselpumpenaggregot nach einem der vorhergehenden Ansprüche, **dadurch gekennzeichnet, dass** die Steuerelektronik (10, 11, 16) so ausgebildet ist, dass sie den Antrieb (2) der Pumpe (1) umsteuert, insbesondere kavitierend ansteuert, sobald mittels der Vorrichtung (20 - 22) eine Anzahl von Partikeln pro Volumeneinheit der Förderflüssigkeit und/oder eine Art von Partikeln erfasst wird, die einen zuvor festgelegten Wert erreicht und/oder einer zuvor festgelegten Art entspricht.

7. Kreiselpumpenaggregat nach einem der vorhergehenden Ansprüche, **dadurch gekennzeichnet, dass** die Steuerelektronik (10, 11, 16) so ausgebildet ist, dass sie in vorbestimmten zeitlichen Abstand durch öffnende Ventilansteuerung der Vorrichtung (20 - 22) eine Flüssigkeitsmenge vom Förderstrom zuführt und die Vorrichtung (20 - 22) zum Erfassen von Partikeln ansteuert.

8. Kreiselpumpenaggregat nach einem der vorhergehenden Ansprüche, **dadurch gekennzeichnet, dass** die Vorrichtung (20 - 22) einen Probenträger aufweist, der von einer Gehäuseseite zugänglich und auswechselbar angeordnet ist.

9. Kreiselpumpenaggregat nach einem der vorhergehenden Ansprüche, **dadurch gekennzeichnet, dass** zumindest der mechanische/optische Teil der Vorrichtung (20 - 22) schwingungsgedämpft innerhalb des Gehäuses (8) angeordnet ist.

10. Kreiselpumpenaggregat nach einem der vorhergehenden Ansprüche, **dadurch gekennzeichnet, dass** eingangsseitig der Vorrichtung Mittel (17) zur Druckreduzierung vorgesehen sind.

11. Kreiselpumpenaggregot nach Anspruch 10, **dadurch gekennzeichnet, dass** die Mittel (17) zur Druckreduzierung zum Zwecke des Spülens deaktivierbar sind.

12. Kreiselpumpenaggregot nach einem der vorhergehenden Ansprüche, **dadurch gekennzeichnet, dass** die Vorrichtung eine vorzugsweise im Elektronikgehäuse (8) angeordnete Pumpe (18) zum Zuführen und/oder Abführen von Förderflüssigkeit aufweist.

13. Kreiselpumpenaggregot nach einem der vorhergehenden Ansprüche, **dadurch gekennzeichnet, dass** die Vorrichtung eine vorzugsweise im Elektronikgehäuse (8) angeordnete Bestrahlungseinrichtung (24) für die in der Vorrichtung (20 - 22) befindliche oder aus der Vorrichtung (20 - 22) herausgeführte Flüssigkeit aufweist.

14. Kreiselpumpenaggregot nach einem der vorhergehenden Ansprüche, **dadurch gekennzeichnet, dass** die Steuerelektronik die Steuer- und Auswertelektronik (16) der Vorrichtung (20 - 22) sowie eine Steuerelektronik (11) der Pumpe, vorzugsweise eine Drehzahlstellerelektronik (10) umfasst.

15. Kreiselpumpenaggregot nach einem der vorhergehenden Ansprüche, **dadurch gekennzeichnet, dass** die Steuerelektronik Mittel zum Speichern von Daten der Auswertelektronik sowie Mittel (15) zum drahtgebundenen und/oder drahtlosen Übertragen von Daten der Auswertelektronik und/oder von gespeicherten Daten umfasst.

16. Verfahren zum Ansteuern eines drehzahlsteuerbaren Kreiselpumpenaggregats, insbesondere zur Trinkwasserförderung, bei welchem die Förderflüssigkeit quantitativ und/oder qualitativ hinsichtlich der darin befindlichen Partikel, insbesondere Bakterien, überwacht wird, **dadurch gekennzeichnet, dass** die Drehzahl in Abhängigkeit der Art und/oder Anzahl der erfassten Partikel erfolgt.

17. Verfahren nach Anspruch 16, **dadurch gekennzeichnet, dass** beim Erreichen einer Anzahl von Partikeln pro Volumeneinheit der Förderflüssigkeit die Pumpe zeitweise mit erhöhter Drehzahl angesteuert wird.

18. Verfahren nach Anspruch 16 oder 17, dass beim Erreichen einer Anzahl von Partikeln pro Volumeneinheit der Förderflüssigkeit und/oder beim Erfassen einer vorbestimmten Art von Partikeln die Pumpe zur Erzeugung von Kavitation angesteuert wird.
